**Europäisches Patentamt**

**European Patent Office**

**Office européen des brevets**

(19)

(11) Veröffentlichungsnummer : **0 365 467 B1**

(12) **EUROPÄISCHE PATENTSCHRIFT**

(45) Veröffentlichungstag der Patentschrift :
**18.08.93 Patentblatt 93/33**

(51) Int. Cl.⁵ : **A61K 49/00**

(21) Anmeldenummer : **89730095.0**

(22) Anmeldetag : **06.04.89**

(54) **Ultraschallkontrastmittel aus Gasbläschen und Fettsäure-enthaltenden Mikropartikeln.**

(30) Priorität : **07.10.88 DE 3834705**

(43) Veröffentlichungstag der Anmeldung :
**25.04.90 Patentblatt 90/17**

(45) Bekanntmachung des Hinweises auf die
Patenterteilung :
**18.08.93 Patentblatt 93/33**

(84) Benannte Vertragsstaaten :
**AT BE CH DE ES FR GB GR IT LI LU NL SE**

(56) Entgegenhaltungen :
**EP-A- 0 122 624**
**EP-A- 0 123 235**
**DE-C- 3 637 926**

(73) Patentinhaber : **SCHERING
AKTIENGESELLSCHAFT Berlin und
Bergkamen
Müllerstrasse 170/178 Postfach 65 03 11
D-13303 Berlin (DE)**

(72) Erfinder : **Lange, Lothar, Prof.
Beskidenstrasse 24
D-1000 Berlin 38 (DE)**
Erfinder : **Hilmann, Jürgen
Ugandastrasse 9
D-1000 Berlin 65 (DE)**
Erfinder : **Fritzsch, Thomas, Dr.
Elisenstrasse 2
D-1000 Berlin 41 (DE)**
Erfinder : **Siegert, Joachim, Dr.
Weddingstrasse 5
D-1000 Berlin 65 (DE)**

EP 0 365 467 B1

Jouve, 18, rue Saint-Denis, 75001 PARIS

## Beschreibung

Die Erfindung betrifft Kontrastmittel für die Ultraschall-Diagnostik aus Gasbläschen und Mikropartikel, die dadurch gekennzeichnet sind, daß sie als Mikropartikel eine Mischung mindestens einer Fettsäure mit mindestens einem nichtgrenzflächenaktiven Feststoff, suspendiert in einem flüssigen Träger enthalten, wobei als Fettsäure Myristin-, Palmitin-, Stearin- und/oder Arachinsäure und als nichtgrenzflächenaktiver Feststoff Galactose, Fructose, Glucose, Lactose und/oder $\alpha$-Cyclodextrin verwendet werden.

Die Untersuchung von Organen mit Ultraschall (Sonographie) ist eine seit einigen Jahren gut eingeführte und praktizierte diagnostische Methode. Ultraschallwellen im Megahertz-Bereich (oberhalb 2 Mega-Hertz mit Wellenlängen zwischen 1 und 0,2 mm) werden an Grenzflächen von unterschiedlichen Gewebearten reflektiert. Die dadurch entstandenen Echos werden verstärkt und sichtbar gemacht. Von besonderer Bedeutung ist dabei die Untersuchung des Herzens mit dieser Methode die Echokardiographie genannt wird (Haft J.I. et al.; Clinital echocardiography Futura, Mount Kisco, New York 1978; Köhler, E. Klinische Echokardiographie, Enke, Stuttgart 1979; Stefan, G. et al.: Echokardiographie, Thieme, Stuttgart-New York 1981; G. Biamino, L. Lange: Echokardiographie, Hoechst AG, 1983).

Da Flüssigkeiten - auch das Blut - nur dann im Ultraschall-B-Bild Kontrast liefern, wenn Dichte-Unterschiede zur Umgebung bestehen, wurde nach Möglichkeiten gesucht, das Blut und seine Strömung für eine Ultraschall-B-Bild-Untersuchung sichtbar zu machen, was durch die Zugabe von feinsten Gasbläschen auch möglich ist.

Verwendet man bei der Ultraschalluntersuchung des Herzens oder der Gefäße die schwachen Reflexionen des Ultraschalls an den roten Blutkörperchen und nutzt, das sg. Doppler-Phänomen, kann man auch ohne Kontrastmittel die Blutströmung darstellen. Aber auch bei dieser Methode ist die Zugabe von kleinen Gasbläschen in die Blutströmung vorteilhaft weil die stärkeren Reflexionen an den Gasbläschen eine bessere Bildverwertung erlauben [Z Kardiol 77:227-232 (1988)].

Aus der Literatur sind mehrere Methoden zur Herstellung und Stabilisierung der Gasbläschen bekannt. Sie lassen sich beispielsweise vor der Injektion in den Blutstrom durch heftiges Schütteln oder Rühren von Lösungen wie Salzlösungen, Farbstofflösungen oder von vorher entnommenem Blut erzeugen.

Obwohl man dadurch eine Ultraschall-Kontrastgebung erreicht, sind diese Methoden mit schwerwiegenden Nachteilen verbunden, die sich in schlechter Reproduzierbarkeit, stark schwankender Größe der Gasbläschen und - bedingt durch einen Anteil an sichtbaren großen Bläschen - einem gewissen Embolie-Risiko äußern.

Diese Nachteile wurden durch andere Herstellungsverfahren teilweise behoben, wie beispielsweise durch das in US-Patent 3,640,271 beschriebene, bei dem Bläschen mit reproduzierbarer Größe durch Filtration oder durch Anwendung einer unter Gleichstrom stehenden Elektrodenvorrichtung erzeugt werden. Dem Vorteil in der Möglichkeit, Gasbläschen mit reproduzierbarer Größe herstellen zu können, steht der erhebliche technische Aufwand als Nachteil gegenüber.

In dem US-Patent 4,276,885 wird die Herstellung von Gasbläschen mit definierter Größe beschrieben, die mit einer vor dem Zusammenfließen schützenden Gelatine-Hülle umgeben sind. Die Aufbewahrung der fertigen Bläschen kann nur im "eingefrorenen" Zustand erfolgen, beispielsweise durch Aufbewahren bei Kühlschranktemperatur, wobei sie zur Verwendung wieder auf Körpertemperatur gebracht werden müssen.

In dem US-Patent 4,265,251 wird die Herstellung und Verwendung von Gasbläschen mit einer festen Hülle aus Sacchariden beschrieben, die mit einem unter Druck stehenden Gas gefüllt sein können. Stehen sie unter Normaldruck, so können sie als Ultraschallkontrastmittel eingesetzt werden; bei Verwendung mit erhöhtem Innendruck dienen sie der Blutdruckmessung. Obwohl hierbei die Aufbewahrung der festen Gasbläschen kein Problem darstellt, ist der technische Aufwand bei der Herstellung ein erheblicher Kostenfaktor.

Die Risiken der nach dem Stand der Technik zur Verfügung stehenden Kontrastmittel werden durch zwei Faktoren hervorgerufen: Größe und Anzahl sowohl der Feststoffpartikel als auch der Gasbläschen.

Der bisher geschilderte Stand der Technik gestattet die Herstellung von Ultraschallkontrastmitteln, die stets nur einige der geforderten Eigenschaften besitzen:

    1. Ausschalten des Embolierisikos
       - Gasbläschen (Größe und Anzahl)
       - Feststoffpartikel (Größe und Anzahl)
    2. Reproduzierbarkeit
    3. genügend lange Stabilität
    4. Lungengängigkeit, z.B. um Ultraschall-Kontrastierung des linken Herzteiles zu erhalten
    5. Kapillargängigkeit
    6. Sterilität und Pyrogenfreiheit der Zubereitung
    7. leichte Herstellbarkeit mit vertretbarem Kostenaufwand
    8. problemlose Bevorratung.

In der europäischen Patentanmeldung mit der Veröffentlichungs-Nummer 52575 wird zwar die Herstellung von Gasbläschen beschrieben, die diese erforderlichen Eigenschaften besitzen sollen. Zu ihrer Herstellung werden Mikropartikel einer festen kristallinen Substanz, wie beispielsweise Galaktose, in einer Trägerflüssigkleit suspendiert, wobei das Gas, das an der Partikeloberfläche adsorbiert, in Hohlräumen zwischen den Partikel oder in interkristallinen Hohlräumen eingeschlossen ist, die Gasbläschen bildet. Die so entstandene Suspension von Gasbläschen und Mikropartikel wird innerhalb von 10 Minuten injiziert. Obwohl in der europäischen Patentschrift 52575 behauptet wird, daß die nach der beschriebenen Methode hergestellte Suspension geeignet ist, nach Injektion in eine periphere Vene sowohl auf der rechten Herzseite als auch nach Passage der Lunge in der linken Herzseite zu erscheinen und dort das Blut und dessen Strömung bei Ultraschall-Untersuchung sichtbar zu machen, hielt diese Behauptung einer Nachprüfung nicht stand. So wurde festgestellt, daß das nach der in der europäischen Anmeldung 52575 beschriebenen Methode hergestellte und in eine periphere Vene injizierte Kontrastmittel keine Ultraschall-Echos im linken Herzteil erzeugten.

Auch in der EP-A-77752 wird die Herstellung von einer flüssigen Mischung zur Verwendung als Kontrastmittel beschrieben, das wiederum aus einer Mischung eines Tensides oder einer wäßrigen Lösung des Tensides und einer wäßrigen viskosen Trägerflüssigkeit besteht.

In der veröffentlichten europäischen Patentanmeldung (Veröffentlichungs-Nr. 0122624) wird ein Mikropartikel und Gasbläschen enthaltendes Ultraschallkontrast-verstärkendes Mittel beschrieben, das geeignet ist nach intravenöser Applikation und Lungenpassage die Kontrastgebung des linken Herzens, des Myocards sowie anderer Organe, wie der Leber, Milz und Niere zu verstärken. In dieser Anmeldung sind zwar auch Fettsäuren ("gesättigte oder ungesättigte $(C_4$-$C_{20})$-Fettsäuren") als geeignet zur Herstellung der Mikropartikel genannt, beispielhaft belegt sind jedoch nur deren Ester oder Salze als grenzflächenaktive Stoffe wie beispielsweise Ascorbylpalmitat oder Saccharosemonopalmitat. Diese haben jedoch den Nachteil, daß sie in der Formulierung schon bei Lagerung unter Normalbedingungen (25°C) relativ rasch zersetzt werden (s. Tabelle). Dies ist im Hinblick auf ein Handelspräparat und deren Reinheitsforderungen schädlich.

## Tabelle Stabilitätsuntersuchung

Formulierung: A   Galactose + 0,134% Ascorbylpalmitat

B   Galactose + 0,1 % Palmitinsäure

Chemische Stabilität der Additive in Abhängigkeit von der Lagertemperatur und der Zeit

| Lagerdauer | Formulierung | |
|---|---|---|
| | A | B |
| | % (m/m) Ascorbylpalmitat | % (m/m) Palmitinsäure |
| Start | 100% | 100% |
| 6 Wochen | | |
| Raumtemperatur | 84,3% | nicht untersucht |
| 40°C | 67,9% | nicht untersucht |
| 50°C | 33,6% | 97,4% |
| 12 Wochen | | |
| Raumtemperatur | 79,8% | 98,0% |
| 40°C | 53,7% | 98,4% |
| 50°C | 18,7% | 95,1% |

Kontrastintensität:

Mit der Abnahme des Additivgehaltes geht eine Reduktion des Links-Herz-Kontrastes einher.

Der Erfindung liegt somit die Aufgabe zugrunde, Mittel sowie Verfahren zu deren Herstellung zur Verfügung zu stellen, die diesen Nachteil nicht besitzen.

Durch Verwendung der Fettsäuren Myristin-, Palmitin-, Stearin und/oder Arachinsäure als grenzflächen-

3

aktive Substanzen wird diese Aufgabe gelöst.

Die durch Suspendieren der erfindungsgemäßen Mikropartikel in dem flüssigen Träger erhaltenen erfindungsgemäßen Ultraschallkontrastmittel sind in der Lage nach intravenöser Applikation das Blut und dessen Strömungsverhältnisse nicht nur auf der rechten Seite des Herzens sondern auch nach der Passage des Kapilarbettes der Lunge auf der linken Herzseite für Ultraschall sichtbar zu machen.

Darüberhinaus zeigen sie überraschenderweise einen größeren Verstärkungseffekt und bessere Reproduzierbarkeit des Ultraschallkontrasts als die Ultraschallkontrastmittel des Standes der Technik.

Die grenzflächenaktive Substanz in den Mikropartikeln wird in einer Konzentration von 0,01 bis 5 Gewichtsprozent vorzugsweise von 0,04 bis 1 Gewichtsprozent verwendet.

Die Mikropartikel bestehen aus einer Mischung von mindestens einer der genannten grenzflächenaktiven Substanzen mit mindestens einem physiologisch verträglichen nicht grenzflächenaktiven kristallinen Festoff. Wobei als physiologisch verträglicher, nicht grenzflächenaktiver Feststoff Galactose, Fructose, Glucose, Lactose und/oder $\alpha$-Cyclodextrin verwendet werden. Sie sind in einer Konzentration von 95 bis 99,99 Gewichtsprozent in den Mikropartikeln enthalten.

Zur Herstellung der Mikropartikel werden die dafür vorgesehenen Substanzen unter aseptischen Bedingungen rekristallisiert. Anschließend werden sie unter aseptischen Bedingungen zerkleinert, z.B. durch Vermahlung in einer Luftstrahlmühle, bis die gewünschte Partikelgröße erreicht ist. Es wird eine den Erythrocyten vergleichbare Partikelgröße von < 10 $\mu$m, vozugsweise < 8 $\mu$m angestrebt. Der Medianwert des Mikronisates liegt bei 1-3 $\mu$m. Die Partikelgröße wird in geeigneten Meßgeräten bestimmt, Die erzeugten Mikropartikel bestehen aus einer Mischung von grenzflächenaktiver Substanz und nichtgrenzflächenaktivem Feststoff.

Sowohl die durch das Zerkleinerungsverfahren erreichte Größe der Mikropartikel als auch die Größe der im erfindungsgemäßen Kontrastmittel enthaltenen Gasbläschen gewährleistet eine gefahrlose Passage des Kapillarsystems und des Lungenkapillarbettes und schließt das Entstehen von Embolien aus.

Das für die Kontrastgebung benötigte Gas (z.B. Luft, Stickstoff oder Argon) wird durch die Mikropartikel transportiert. Es ist teilweise an der Oberfläche der Mikropartikel absorbiert, in den Hohlräumen zwischen den Mikropartikeln oder interkristallin eingeschlossen.

Das von den Mikropartikeln transportierte Gasvolumen in Form von Gasbläschen beträgt 0,02 bis 0,6 ml pro Gramm Mikropartikel.

Als flüssiger Träger kommen in Frage Wasser, wäßrige Lösungen eines oder mehrerer anorganischer Salze wie physiologische Kochsalzlösung und Pufferlösungen, wäßrige Lösungen von Mono- oder Disacchariden wie Galactose, Glucose oder Lactose, ein- oder mehrwertige Alkohole, soweit sie physiologisch verträglich sind wie Äthanol, Propanol, Isopropylalkohol, Polyethylenglykol, Ethylenglykol, Glycerin, Propylenglykol, Propylenglycolmethylether oder deren wäßrige Lösungen. Bevorzugt sind Wasser und physiologische Elektrolytlösungen wie physiologische Kochsalzlösung sowie wäßrige Zuckerlösungen wie z.B. Galactose, Glucose, Fructose und Lactose. Werden Lösungen verwendet, beträgt die Konzentration des gelösten Stoffes 0,1 bis 30 Gewichtsprozent, vorzugsweise 0,5 bis 15 Gewichtsprozent; insbesondere verwendet man Wasser, 0,9%ige wäßrige Kochsalzlösung oder 5-6%ige wäßrige Galactose-Lösung.

Die Erfindung betrifft auch ein Verfahren zur Herstellung des erfindungsgemäßen Mittels gemäß Anspruch 8.

Zur Herstellung des gebrauchsfertigen Ultrasthall-Kontrastmittels gibt man die sterile Trägerflüssigkeit zu der in Form von Mikropartikel vorliegenden sterilen Kombination mindestens einer der folgenden Fettsäuren Myristin-, Palmitin-, Stearin und/oder Arachinsäure mit mindestens einem der folgenden nichtgrenzflächenaktiven Stoffe Galactose, Fructose, Glucose, Lactose und/oder $\alpha$-Cyclodextrin und schüttelt diese Mischung, bis sich eine homogene Suspension gebildet hat, wofür ca. 5 bis 10 sec. erforderlich sind. Die entstandene Suspension wird sofort nach ihrer Herstellung spätestens jedoch nach 5 Minuten als Bolus in eine periphere Vene oder einen schon vorhandenen Katheter injiziert wobei von 0,01 ml bis 1 ml/kg Körpergewicht appliziert werden.

Aus Gründen der Zweckmäßigkeit werden die zur Herstellung des erfindungsgemäßen Mittels benötigten Komponenten wie Trägerflüssigkeit (A) und Mikropartikel der Kombination Fettsäure und nichtgrenzflächenaktiver Feststoff (B) in der für eine Untersuchung erforderlichen Menge steril in zwei Gefäßen aufbewahrt. Beide Gefäße (Vials) haben Verschlüsse, die die Entnahme und Zugabe mittels Injektionsspritze unter sterilen Bedingungen ermöglichen. Die Größe von Gefäß B ist so beschaffen, daß der Inhalt von Gefäß A mittels Injektionsspritze nach B überführt werden kann und die vereinigten Komponenten geschüttelt werden können.

Die Durchführung einer echokardiographischen Untersuchung an einem 10 kg schweren Pavian soll die Verwendung des erfindungsgemäßen Kontrastmittels demonstrieren:

8,5 ml Trägerflüssigkeit (siehe Herstellungsbeispiele) werden aus einem Vial mit einer Injektionsspritze entnommen und zu 3 g Mikropartikel, die sich in einem zweiten Vial befinden, gegeben und etwa 5 bis 10 sec. geschüttelt, bis sich eine homogene Suspension gebildet hat. Man injiziert 2 ml dieser Suspension in eine periphere Vene (V. jugularis, brachialis oder saphena) über einen 3-WegeHahn mit einer Infusionsgeschwindigkeit von mindestens 1 ml/sec., besser mit 2-3 ml/sec.. An die Kontrastmittelinjektion schließt sich mit derselben Geschwindigkeit sofort die Injektion von 10 ml physiologischer Kochsalzlösüng an damit der Kontrastmittel-Bolus so lange wie möglich erhalten bleibt. Vor während und nach der Injektion wird ein handelsüblicher Schallkopf für die Echokardiographie an den Thorax des Versuchstieres gehalten, so daß ein typischer Querschnitt durch das rechte und linke Herz erhalten wird. Diese Versuchsanordnung entspricht dem Stand der Technik und ist dem Fachmann bekannt.

Erreicht das Ultraschallkontrastmittel das rechte Herz, kann man im 2-D-Echobild oder im M-mode-Echo-Bild verfolgen, wie das durch das Kontrastmittel markierte Blut zunächst die Höhe des rechten Vorhofes, dann die des rechten Ventrikels und der Pulmonalarterie erreicht, wobei für eine für die diagnostische Untersuchung ausreichende Zeit eine homogene Füllung auftritt. Während die Höhlen des rechten Herzens im Ultraschallbild wieder leer werden, erscheint das mit Kontrastmittel markierte Blut nach der Lungenpassage in den Pulmonalvenen wieder, füllt den linken Vorhof, den linken Ventrikel und die Aorta homogen, wobei der Kontrast länger anhält als auf der rechten Herzseite. Neben der Darstellung des Blutflusses durch die Höhlen des linken Herzens kommt es auch zu einer Kontrastierung des Myokards, die die Durchblutung wiederspiegelt.

Die Verwendung des erfindungsgemäßen Ultraschall-Kontrastmittels ist aber nicht beschränkt auf die Sichtbarmachung des Blutstroms im arteriellen Teil des Herzens nach venöser Applikation sondern es wird mit ausgezeichnetem Erfolg auch bei der Untersuchung des rechten Herzens und anderer Organe als Kontrastmittel verwendet.

**BEISPIEL 1**

A) Trägerflüssigkeit: Wasser für Injektionszwecke

B) Herstellung der Mikropartikel:

I 1998 g Galactose werden in 1080 g Wasser gereinigt, gelöst, sterilfiltriert und unter aseptischen Bedingungen auf 6-10°C abgekühlt.

II 2 g Palmitinsäure werden in 120 g Ethanol gelöst, sterilfiltriert und unter Rühren I zugesetzt.

III Die vereinigten Lösungen werden unter aseptischen Bedingungen bei ca. 40°C und 50 mbar Unterdruck zur Trockne gebracht.

IV Das Rekristallisat wird unter aseptischen Bedingungen mit einer Luftstrahlmühle auf folgende Korngrößenverteilung zerkleinert:

$$D_{10} \leqq 1 \ \mu m$$
$$D_{50} \leqq 2,5 \ \mu m$$
$$D_{90} \leqq 5 \ \mu m$$

Die Bestimmung der Korngrößenverteilung erfolgt nach Suspendierung des Mikronisates in Alkohol mit einem Partikelmeßgerät (z.B. Cilas-Granulometer 715).

V Die Abpackung der Mikropartikel erfolgt in 20 ml Vials zu jeweils 3 g.

C) Herstellung des gebrauchsfertigen Ultraschall-Kontrastmittels

In das 20 ml-Vial, welches 3 g Mikropartikel enthält, werden mittels Injektionspritze 8,5 ml Wasser für Injektionszwecke transferiert und bis zum Entstehen einer homogenen Suspension geschüttelt (5-10 Sekunden).

**BEISPIEL 2**

A) Trägerflüssigkeit: Wasser für Injektionszwecke

B) Herstellung der Mikropartikel:

I 1998 g Galactose werden in 1080 g Wasser gereinigt, gelöst, sterilfiltriert und unter aseptischen Bedingungen auf 6-10°C abgekühlt.

II 2 g Myristinsäure werden in 120 g Ethanol gelöst, sterilfiltriert und unter Rühren I zugesetzt.

III Die vereinigten Lösungen werden unter aseptischen Bedingungen bei ca. 40°C und 50 mbar Unterdruck zur Trockne gebracht.

IV Das Rekristallisat wird unter aseptischen Bedingungen mit einer Luftstrahlmühle auf folgende Korngrößenverteilung zerkleinert:

$D_{10} \leqq 1 \ \mu m$
$D_{50} \leqq 2,5 \ \mu m$
$D_{90} \leqq 5 \ \mu m$

Die Bestimmung der Korngrößenverteilung erfolgt nach Suspendierung des Mikronisates in Alkohol mit einem Partikelmeßgerät (z.B. Cilas-Granulometer 715).

V Die Abpackung der Mikropartikel erfolgt in 20 ml Vials zu jeweils 3 g.

C) Herstellung des gebrauchsfertigen Ultraschall-Kontrastmittels

In das 20 ml-Vial, welches 3 g Mikropartikel enthält, werden mittels Injektionspritze 8,5 ml Wasser für Injektionszwecke transferiert und bis zum Entstehen einer homogenen Suspension geschüttelt (5-10 Sekunden).

**BEISPIEL 3**

A) Trägerflüssigkeit: Wasser für Injektionszwecke

B) Herstellung der Mikropartikel:

I 1998 g Galactose werden in 1080 g Wasser gereinigt, gelöst, sterilfiltriert und unter aseptischen Bedingungen auf 6-10°C abgekühlt.

II 2 g Stearinsäure werden in 120 g Ethanol gelöst, sterilfiltriert und unter Rühren I zugesetzt.

III Die vereinigten Lösungen werden unter aseptischen Bedingungen bei ca. 40°C und 50 mbar Unterdruck zur Trockne gebracht.

IV Das Rekristallisat wird unter aseptischen Bedingungen mit einer Luftstrahlmühle auf folgende Korngrößenverteilung zerkleinert:

$D_{10} \leqq 1 \ \mu m$
$D_{50} \leqq 2,5 \ \mu m$
$D_{90} \leqq 5 \ \mu m$

Die Bestimmung der Korngrößenverteilung erfolgt nach Suspendierung des Mikronisates in Alkohol mit einem Partikelmeßgerät (z.B. Cilas-Granulometer 715).

V Die Abpackung der Mikropartikel erfolgt in 20 ml Vials zu jeweils 3 g.

C) Herstellung des gebrauchsfertigen Ultraschall-Kontrastmittels

In das 20 ml-Vial, welches 3 g Mikropartikel enthält, werden mittels Injektionspritze 8,5 ml Wasser für Injektionszwecke transferiert und bis zum Entstehen einer homogenen Suspension geschüttelt (5-10 Sekunden).

**BEISPIEL 4**

A) Trägerflüssigkeit: Wasser für Injektionszwecke

B) Herstellung der Mikropartikel:

I 1998 g Galactose werden in 1080 g Wasser gereinigt, gelöst, sterilfiltriert und unter aseptischen Bedingungen auf 6-10°C abgekühlt.

II 1 g Myristinsäure + 1 g Arachinsäure werden in 120 g Ethanol gelöst, sterilfiltriert und unter Rühren I zugesetzt.

III Die vereinigten Lösungen werden unter aseptischen Bedingungen bei ca. 40°C und 50 mbar Unterdruck zur Trockne gebracht.

IV Das Rekristallisat wird unter aseptischen Bedingungen mit einer Luftstrahlmühle auf folgende Korngrößenverteilung zerkleinert:

$D_{10} \leqq 1 \ \mu m$
$D_{50} \leqq 2,5 \ \mu m$
$D_{90} \leqq 5 \ \mu m$

Die Bestimmung der Korngrößenverteilung erfolgt nach Suspendierung des Mikronisates in Alkohol mit einem Partikelmeßgerät (z.B. Cilas-Granulometer 715).

V Die Abpackung der Mikropartikel erfolgt in 20 ml Vials zu jeweils 3 g.

C) Herstellung des gebrauchsfertigen Ultraschall-Kontrastmittels

In das 20 ml-Vial, welches 3 g Mikropartikel enthält, werden mittels Injektionspritze 8,5 ml Wasser für Injektionszwecke transferiert und bis zum Entstehen einer homogenen Suspension geschüttelt (5-10 Sekunden).

**BEISPIEL 5**

A) Herstellung der Trägerflüssigkeit:

55 g Galaktose werden in Wasser p.i. gelöst, bis zu einem Volumen von 1000 ml aufgefüllt, durch ein 0,2 μm Filter filtriert, jeweils 10 ml der filtrierten Lösung in 10 ml Vials abgefüllt und 15 Minuten bei 121°C sterilisiert.

B) Herstellung der Mikropartikel:

I 1998 g Galactose werden in 1080 g Wasser gereinigt, gelöst, sterilfiltriert und unter aseptischen Bedingungen auf 6-10°C abgekühlt.
II 1 g Palmitinsäure + 1 g Stearinsäure werden in 120 g Ethanol gelöst, sterilfiltriert und unter Rühren 1 zugesetzt.
III Die vereinigten Lösungen werden unter aseptischen Bedingungen bei ca. 40°C und 50 mbar Unterdruck zur Trockne gebracht.
IV Das Rekristallisat wird unter aseptischen Bedingungen mit einer Luftstrahlmühle auf folgende Korngrößenverteilung zerkleinert:
$$D_{10} \leqq 1 \ \mu m$$
$$D_{50} \leqq 2,5 \ \mu m$$
$$D_{90} \leqq 5 \ \mu m$$
Die Bestimmung der Korngrößenverteilung erfolgt nach Suspendierung des Mikronisates in Alkohol mit einem Partikelmeßgerät (z.B. Cilas-Granulometer 715).
V Die Abpackung der Mikropartikel erfolgt in 20 ml Vials zu jeweils 3 g.

C) Herstellung des gebrauchsfertigen Ultraschall-Kontrastmittels

In das 20 ml-Vial, welches 3 g Mikropartikel enthält, werden mittels Injektionspritze 8,5 ml Galaktoselösung A transferiert und bis zum Entstehen einer homogenen Suspension geschüttelt (5-10 Sekunden).

**Patentansprüche**

1. Kontrastmittel für die Ultraschalldiagnostik aus Gasbläschen und Mikropartikeln, bestehend aus einem Gemisch aus mindestens einem nicht grenzflächenaktiven Feststoff und einer Fettsäure, suspendiert in einem flüssigen Träger, dadurch gekennzeichnet, daß die Mikropartikel als Fettsäure Myristin-, Palmitin-, Stearrinund/oder Arachinsäure in einer Konzentration von 0,01 bis 5 Gewichtsprozent und als nicht grenzflächenaktiven Feststoff Galactose, Fructose, Glucose, Lactose und/oder α-Cyclodextrin enthalten.

2. Mittel nach Anspruch 1, dadurch gekennzeichnet, daß es Mikropartikel enthält, die Myristin-, Palmitin-, Stearrin- und/oder Arachinsäure in einer Konzentration von 0,04 bis 1 Gewichtsprozent enthalten.

3. Mittel nach Anspruch 1, dadurch gekennzeichnet, daß es als physiologisch verträglichen flüssigen Träger Wasser, physiologische Elektrolytlösung, die wäßrige Lösung von ein- oder mehrwertigen Alkoholen wie Glycerin, Polyethylenglycol oder von Propylenglycolmethylether oder die wäßrige Lösung eines Mono- oder Disaccharides enthält.

4. Mittel nach Anspruch 1 und 6, dadurch gekennzeichnet, daß es als physiologisch verträglichen flüssigen Träger Wasser, physiologische Kochsalzlösung oder 5 bis 6 %ige wäßrige Galactoselösung enthält.

5. Mittel nach Anspruch 1, dadurch gekennzeichnet, daß es Mikropartikel aus einer Mischung von Palmitinsäure und Galactose, suspendiert in Wasser, enthält.

6. Mittel nach Anspruch 1, dadurch gekennzeichnet, daß es Mikropartikel aus einer Mischung von Myristinsäure und Galactose, suspendiert in Wasser, enthält.

7. Mittel nach Anspruch 1, dadurch gekennzeichnet, daß es Mikropartikel aus einer Mischung von Stearrinsäure und von Galactose, suspendiert in Wasser, enthält.

8. Verfahren zur Herstellung eines Mikropartikel und Gasbläschen enthaltenden Kontrastmittels für die Ultraschalldiagnostik, dadurch gekennzeichnet, daß man Mikropartikel bestehend aus Myristin-, Palmitin-, Stearrin- und/oder Arachinsäure und Galactose, Fructose, Glucose, Lactose und/oder α-Cyclodextrin mit einer Trägerflüssigkeit vereinigt und bis zum Entstehen einer homogenen Suspension schüttelt.

## Claims

1. Contrast medium for ultrasound diagnostics, comprising gas bubbles and microparticles comprising a mixture of at least one non-surface-active solid and a fatty acid, suspended in a liquid carrier, characterised in that the microparticles contain as fatty acid myristic, palmitic, stearic and/or arachidic acid at a concentration of from 0.01 to 5 % by weight and, as non-surface-active solid, galactose, fructose, glucose, lactose and/or α-cyclodextrin.

2. Medium according to claim 1, characterised in that it contains microparticles that contain myristic, palmitic, stearic and/or arachidic acid at a concentration of from 0.04 to 1 % by weight.

3. Medium according to claim 1, characterised in that it contains as physiologically tolerable liquid carrier water, physiological electrolyte solution, an aqueous solution of a monohydric or polyhydric alcohol, such as glycerol, polyethylene glycol or propylene glycol methyl ether, or an aqueous solution of a mono- or di-saccharide.

4. Medium according to claims 1 and 6, characterised in that it contains as physiologically tolerable liquid carrier water, physiological saline solution or from 5 to 6 % aqueous galactose solution.

5. Medium according to claim 1, characterised in that it contains microparticles of a mixture of palmitic acid and galactose, suspended in water.

6. Medium according to claim 1, characterised in that it contains microparticles of a mixture of myristic acid and galactose, suspended in water.

7. Medium according to claim 1, characterised in that it contains microparticles of a mixture of stearic acid and galactose, suspended in water.

8. Process for the preparation of a contrast medium comprising microparticles and gas bubbles for ultrasound diagnostics, characterised in that microparticles comprising myristic, palmitic, stearic and/or arachidic acid and galactose, fructose, glucose, lactose and/or α-cyclodextrin are combined with a carrier liquid and the whole is shaken until a homogeneous suspension has formed.

## Revendications

1. Agent de contraste pour des diagnostics par ultrasons, c'est-à-dire par échographie, formé de microbulles gazeuses et de microparticules elles-mêmes constituées d'un mélange d'une ou de plusieurs matières solides non-surfactives et d'un ou de plusieurs acides gras, en suspension dans un véhicule liquide, agent caractérisé en ce que les microparticules comprennent comme acides gras l'acide myristique, palmitique, stéarique ou arachidique ou plusieurs de ces acides à la fois à une concentration de 0,01 à 5 % en poids, et comme matières non-surfactives du galactose, du fructose, du glucose, du lactose et/ou de l'α-cyclodextrine.

2. Agent selon la revendication 1, caractérisé en ce que ses microparticules contiennent les acides gras indiqués à une concentration de 0,04 à 1 % en poids.

3. Agent selon la revendication 1 dont le véhicule liquide physiologiquement compatible est de l'eau, une solution d'électrolyte physiologique, une solution aqueuse d'un monoalcool ou d'un polyalcool comme le glycérol, un polyéthylène-glycol ou encore l'éther méthylique de propylène-glycol, ou une solution aqueuse d'un monosaccharide ou d'un disaccharide.

4. Agent selon la revendication 1, caractérisé en ce que son véhicule liquide physiologiquement compatible est de l'eau, du sérum physiologique ou une solution aqueuse de 5 à 6 % de galactose.

5. Agent selon la revendication 1, caractérisé en ce qu'il contient des microparticules d'un mélange d'acide palmitique et de galactose en suspension dans de l'eau.

6. Agent selon la revendication 1, caractérisé en ce qu'il contient des microparticules d'un mélange d'acide myristique et de galactose en suspension dans de l'eau.

7. Agent selon la revendication 1, caractérisé en ce qu'il contient des microparticules d'un mélange d'acide stéarique et de galactose en suspension dans de l'eau.

8. Procédé de préparation d'un agent de contraste comprenant des microparticules et des microbulles gazeuses pour des diagnostics par ultrasons, procédé caractérisé en ce que l'on mélange des microparticules formées d'acide myristique, palmitique, stéarique et/ou arachidique, et de galactose, fructose, glucose, lactose et/ou α-cyclodextrine, avec un véhicule liquide, et on secoue le mélange jusqu'à ce qu'il se soit formé une suspension homogène.